# EUROPÄISCHE PATENTSCHRIFT

(11) **EP 2 332 500 B1**
(45) Veröffentlichungstag und Bekanntmachung des Hinweises auf die Patenterteilung: **04.07.2012**
(21) Anmeldenummer: 09015184.6
(22) Anmeldetag: 08.12.2009
(51) Int. Cl.: A61F 5/41

(54) **Vorrichtung zur Penisextension**
Penis extension device
Dispositif d'extension de pénis

(43) Veröffentlichungstag der Anmeldung: 15.06.2011
(73) Patentinhaber: Iptoserv GmbH, 40237 Düsseldorf (DE)
(72) Erfinder:
(74) Vertreter: Stenger, Watzke & Ring

(56) Entgegenhaltungen:
- WO-A1-2008/059566
- CH-A- 347 300
- DE-U1- 9 105 928
- US-A- 4 175 554
- US-A- 5 669 869

## Beschreibung

Die Erfindung betrifft eine Vorrichtung zur Penisextension durch langzeitliche Dehnungsbehandlung, mit einem einen evakuierbaren Volumenraum bereitstellenden Schlauch- oder Rohrkörper.

Penisextensionsgeräte im Allgemeinen und auch im Speziellen solche, die mit Vakuum arbeiten, sind aus dem Stand der Technik an sich bekannt.

Obgleich sich vorbekannte Vorrichtungen zur Penisextension auf dem Markt befinden, sind diese mit einer Reihe von Nachteilen verbunden, die eine wirkungsvolle Anwendung nahezu ausschließen, weshalb insofern Verbesserungsbedarf besteht. Es ist insbesondere erwünscht, die Anwendung über einen längeren Zeitraum zu ermöglichen, sie zu vereinfachen und den Tragekomfort zu verbessern.

Eine Vorrichtung gemäß des Oberbegriffes von Anspruch 1 ist aus der Patentschrift WO 2008/059566 bekannt.

Es ist deshalb die **Aufgabe** der Erfindung eine Vorrichtung zur Penisextension bereitzustellen, die bei gleichzeitig verbessertem Tragekomfort eine vereinfachte Anwendung ermöglicht.

Zur **Lösung** dieser Aufgabe wird mit der Erfindung eine Vorrichtung zur Penisextension der eingangs genannten Art vorgeschlagen, die gekennzeichnet ist durch eine der Penisaufnahme dienende Kompressionshülle, die als innenliegende Hülle innerhalb des vom Schlauch- oder Rohrkörper bereitgestellten Volumenraums angeordnet ist.

Die erfindungsgemäße Vorrichtung verfügt über einen vorzugsweise leichten, flexiblen und knickfesten Schlauch- oder Rohrkörper einerseits und eine Kompressionshülle andererseits. Dabei ist die Kompressionshülle innerhalb des vom Schlauch- oder Rohrkörper bereitgestellten Volumenraums angeordnet.

Die Kompressionshülle dient der eigentlichen Penisaufnahme. Sie ist aus einem dehnungsfähigen Material wie zum Beispiel Gummi, Kautschuk, Silikon und/oder dergleichen Material gebildet. Im bestimmungsgemäßen Anwendungsfall nimmt sie den Penis vollständig auf und sorgt bei einem innerhalb des Schlauch- oder Rohrkörpers angelegten Vakuum dafür, dass nicht vorrangig die Haut, sondern die im Penis befindlichen Schwellkörper und Blutgefäße gedehnt werden. Infolge dieser Beanspruchung kommt es zu schmerzfreien Mikrofaserrissen, in die im Zuge der Ausheilung organismusseitig neues Zellmaterial eingebaut wird. In der Konsequenz ergibt sich eine Penisextension sowohl in Längsrichtung als auch hinsichtlich des Umfangs. Dabei kann in vorteilhafter Weise mittels einer auf den jeweiligen Anwendungsfall anatomisch angepassten Kompressionshülle festgelegt werden, ob eine stärkere Expansion des Penis der Länge oder des Umfangs nach erfolgen soll.

Der Schlauch- oder Rohrkörper weist einen beidseitig offenen, flexiblen Schlauch- oder Rohrabschnitt auf. Dieser ist mittels eines entsprechenden Verschlusselements einendseitig vakuumdicht verschließbar. Anderendseitig verfügt der Schlauch- oder Rohrabschnitt über einen verstärkt ausgebildeten Ringabschnitt, der der vorzugsweise auswechselbaren Anordnung der Kompressionshülle dient.

Zum Zwecke der Evakuierung des vom Schlauch- oder Rohrkörper bereitgestellten Volumenraums verfügt entweder das Verschlusselement oder der Ringabschnitt über einen Anschlussstutzen, der dem strömungstechnischen Anschluss einer vorzugsweise elektrischen, über Akkus betriebenen, mobilen Vakuumpumpe dient. Der Schlauch- oder Rohrabschnitt des Schlauch- oder Rohrkörpers ist flexibel ausgebildet und beispielsweise aus einem Wellen- oder Faltenschlauch gebildet.

Der Schlauch- oder Rohrkörper entspricht in Umfang und Länge ca. 120 % des erigierten Penis. Somit wird einer möglichen Verletzungsgefahr bei einer nächtlichen Erektion vorgebeugt. Die Kompressionshülle weist indes eine Länge und einen Umfang auf, die der Länge und dem Umfang eines Penis im schlaffen Zustand entspricht.

Im bestimmungsgemäßen Anwendungsfall wird ein Penis unter Ausbildung eines Vakuums im Schlauch- oder Rohrkörper in die Kompressionshülle eingesogen, gegebenenfalls unter Zuhilfenahme von Gleitgel. Infolge des anliegenden Vakuums saugt sich der Schlauch- oder Rohrkörper am Penisschaft fest. Es wird so ein lagestabiler und sicherer Halt der gesamten erfindungsgemäßen Vorrichtung erreicht.

Durch den dauerhaft vom Schlauch- oder Rohrkörper bereitgestellten Unterdruck mittels einer portablen, akkubetriebenen Vakuumpumpe kommt es bei kontinuierlicher Anwendung zu einer dauerhaften Dehnung des Penisgewebes hinsichtlich Umfang und Länge, wobei es aufgrund der anliegenden Kompressionshülle insbesondere zu einer Dehnungsbelastung der im Penis befindlichen Schwellkörper und Blutgefäße kommt. Als Unterdruck kann beispielsweise ein Druck im Bereich von 0,5 bar bis 0,9 bar, vorzugsweise von 0,75 bar angelegt werden.

Der Vorteil der erfindungsgemäßen Vorrichtung ergibt sich insbesondere dadurch, dass aufgrund der Hohlkörper-Hüllen-Konstruktion ein verhältnismäßig geringer Unterdruck ausreichend ist, um eine ordnungsgemäße und dauerhaft sichere Haftung am Körper zu gewährleisten. Aus dem Stand der Technik vorbekannte Vakuumvorrichtungen, die mit einem starren und schweren Zylinder sowie ohne Kompressionshülle arbeiten, bedarf es im Unterschied zur erfindungsgemäßen Ausgestaltung eines sehr viel höheren Unterdrucks. Derartige Vorrichtungen können deshalb auch nur über einen weitaus kürzeren Zeitraum eingesetzt werden und bergen darüber hinaus insbesondere die Risiken von Blutergüssen, Blasenbildung und Thrombosen am und im Penis sowie einer Überdehnung oder Verletzung des Penisgewebes. Darüber hinaus führen vorbekannte Vorrichtungen in nachteiliger Weise zu einer gesundheitlich unbedenklichen, aber ästhetisch unansehnlichen Wasseransammlung im Gewebe (Lymphstau). Dieser nachteilige Effekt tritt bei einer Verwendung der erfindungsgemäßen Vorrichtung in vorteilhafter Weise nicht auf. Eine Tragedauer von acht Stunden pro Tag lässt die erfindungsgemäße Vorrichtung ohne weiteres zu.

Der Schlauch- oder Rohrkörper nach der Erfindung ist aus einem leichten Kunststoffmaterial gebildet, das flexibel und gleichzeitig knickstabil geformt ist. Diese Materialwahl wurde getroffen, weil es im Unterschied zu vorbekannten Vorrichtungen aus beispielsweise Glas oder Hartkunststoff nicht der Anlegung eines vergleichsweise großen Unterdrucks bedarf. Die erfindungsgemäße Vorrichtung kann deshalb auch in vorteilhafter Weise nachts während des Schlafes oder tagsüber diskret unter der Kleidung getragen werden, was aus dem Stand der Technik vorbekannte Vorrichtungen nicht ermöglichen. Im Ergebnis erlaubt die erfindungsgemäße Vorrichtung eine im Unterschied zum Stand der Technik längere Anwendungsdauer pro Tag, was sich wiederum in vorteilhafter Weise in einer gesteigerten Wirksamkeit niederschlägt.

Durch eine entsprechende Größenwahl des Hohlkörpers und der Kompressionshülle kann diese an anatomische Gegebenheiten angepasst werden, was im Unterschied zu aus dem Stand der Technik vorbekannten Vorrichtungen auch die Aufnahme von Mikropenissen gestattet.

Die erfindungsgemäße Ausgestaltung verfügt aufgrund ihrer Konstruktion über ein vergleichsweise geringes Gewicht. Sie wird mit einem im Vergleich zum Stand der Technik geringeren Unterdruck betrieben, wobei aber aufgrund des vergleichsweise geringen Gewichts eine längere tägliche Anwendungsdauer möglich ist. Dabei schont die anatomisch angepasste Kompressionshülle das Penisgewebe, so dass Überdehnungen der Haut, Gefäßverletzungen, Blutergüsse oder die Ansammlung vom Lymphflüssigkeit ausgeschlossen werden können. Darüber hinaus bleibt die Bewegungsfreiheit nahezu uneingeschränkt. So ist selbst die Bauchlage während des Schlafes möglich.

Aufgrund des vergleichsweise geringen Vakuums werden im Übrigen ästhetisch unansehnliche Druckstellen und Verformungen des Penis vermieden, wie sie bei Vorrichtungen auftreten können, die mit starkem Zug arbeiten oder bei denen eine Umschlingung der Penisspitze erfolgt, was zu einer Unterbrechung der Blutzufuhr führen kann. Dabei ist aufgrund der Flexibilität der Kompressionshülle und der Größe des Schlauch- oder Rohrkörpers eine nächtliche Peniserektion unproblematisch.

Die erfindungsgemäße Konstruktion gestattet eine Kombination der einzelnen Baukomponenten nach dem Baukastenprinzip. So können in der Ausgestaltung unterschiedliche Schlauch- oder Rohrkörper mit unterschiedlich ausgestalteten Kompressionshüllen wahlweise kombiniert werden. Die Anpassung an anatomische Gegebenheiten ist so in einfacher Weise möglich.

Die Kompressionshülle ist mit dem Schlauch- oder Rohrkörper in einfacher Weise auswechselbar verbunden. Als Verbrauchsartikel kann die Kompressionshülle nach einiger Anwendungszeit ausgetauscht und durch eine andere Kompressionshülle ersetzt werden. Damit wird auch dem Hygieneanspruch Rechnung getragen. Ferner ist hierdurch eine Anpassung an das mittels der Vorrichtung bewirkte Peniswachstum möglich. Alternativ kann auch vorgesehen sein, den Schlauch- oder Rohrkörper zusammen mit der Kompressionshülle integrativ als Verbrauchsartikel auszubilden.

Die erfindungsgemäße Vorrichtung dient dazu, den Leidensdruck aufgrund subjektiv empfundener oder tatsächlich vorhandener Penishypoplasie durch eine vollkommen schmerzlose, unkomplizierte, risikoarme und kostengünstige Penisextension in Länge und Umfang zu beseitigen. Insbesondere gestattet die erfindungsgemäße Vorrichtung die Behandlung intersexueller Männer schon ab dem frühen Jugendalter, denn sie hilft beim Mikropenis durch vollkommen schmerzlose kontinuierliche Dehnung eine objektive Verlängerung und Verdickung desselben zu erzielen. Darüber hinaus kann die efindungsgemäße Vorrichtung bei Patienten mit Prostatakrebs nach einer Operation helfen, den durch Hormonentzug verkleinerten Penis zu vergrößern.

Der Schlauch- oder Rohrkörper sowie die Kompressionshülle können nach Länge und Umfang dem Grunde nach beliebig ausgebildet sein. Die Anpassung an anatomische Gegebenheiten ist deshalb in vorteilhafter Weise möglich. Dabei weist der Schlauch- oder Rohrkörper penisöffnungseingangsseitig, das heißt schaftseitig in einer alternativen Ausgestaltung der Erfindung eine Abschrägung auf. Im bestimmungsgemäßen Verwendungsfall lässt sich der von der Vorrichtung aufgenommene Penis frei in alle Richtungen bewegen und lagern. Dies ermöglicht ein Tragen der erfindungsgemäßen Vorrichtung unter normaler Kleidung oder während des Schlafes. Die Behandlung findet ohne Schmerzen und Einschränkungen statt und kann über viele Stunden zu jeder Zeit durchgeführt werden. Als Folge einer konsequenten Anwendung der erfindungsgemäßen Vorrichtung über täglich mehrere Stunden tritt eine dauerhafte Vergrößerung des gesamten Penis inklusive der Eichel bereits nach wenigen Monaten ein.

Weitere Vorteile und Merkmale der Erfindung ergeben sich aus der nachfolgenden Beschreibung anhand der Fign. Dabei zeigen
- Fig. 1: in einer schematischen Darstellung die erfindungsgemäße Vorrichtung in einer ersten Ausführungsform gemäß einer ersten Anwendungssituation;
- Fig. 2: in einer schematischen Darstellung die erfindungsgemäße Vorrichtung in einer ersten Ausführungsform gemäß einer zweiten Anwendungssituation;
- Fig. 3: in einer schematischen Darstellung die erfindungsgemäße Vorrichtung in einer zweiten Ausführungsform und
- Fig. 4: in einer schematischen Darstellung die erfindungsgemäße Vorrichtung in einer dritten Ausführungsform.

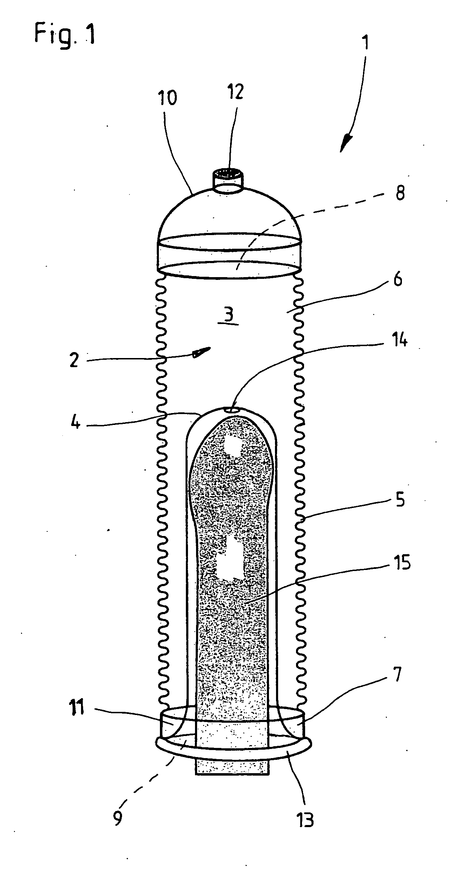

Die erfindungsgemäße Vorrichtung 1 zur Penisextrusion ist in einer ersten Ausführungsform in Fig. 1 dargestellt.

Die erfindungsgemäße Vorrichtung verfügt über einen Schlauch- oder Rohrkörper 2 einerseits und eine Kompressionshülle 4 andererseits. Der Schlauch- oder Rohrkörper 2 stellt einen evakuierbaren Volumenraum 3 zur Verfügung. Dieser Volumenraum 3 nimmt die Kompressionshülle 4 als quasi innere Hülle auf. Der Schlauch- oder Rohrkörper 2 ist im Querschnitt vorzugsweise kreisförmig ausgebildet. Es kommt aber auch eine andere Querschnittsausgestaltung in Frage, beispielsweise eine ovale.

Der Schlauch- oder Rohrkörper 2 verfügt über einen Schlauch- oder Rohrabschnitt 5, der beidendseitig offen ausgebildet ist. Dabei stellt das erste Ende 6 eine erste Öffnung 8 und das zweite Ende 7 eine zweite Öffnung 9 bereit.

Die erste Öffnung 8 ist mittels eines Verschlusselements 10 vakuumdicht verschlossen. Im gezeigten Ausführungsbeispiel ist das Verschlusselement 10 haubenartig ausgestaltet und stellt einen Anschlussstutzen 12 bereit. Dieser Anschlussstutzen 12 dient dem Anschluss einer Vakuumpumpe, gegebenenfalls unter Zwischenschaltung eines Schlauches.

Die zweite Öffnung 9, das heißt die Einführungsöffnung für den Penis 15 verfügt über einen verstärkt ausgebildeten Ringabschnitt 11. Die innerhalb des Volumenraums 3 des Schlauch- oder Rohrkörpers 2 angeordnete Kompressionshülle 4 umgreift mit einer Ringwulst 13 den Ringabschnitt 11, so dass eine lösbare, gleichwohl aber im bestimmungsgemäßen Verwendungsfall sichere Anordnung der Kompressionshülle 4 am Schlauch- oder Rohrabschnitt 5 ausgebildet ist. Es kann alternativ auch vorgesehen sein, Kompressionshülle 4 und Schlauch- oder Rohrkörper integrativ, das heißt als integrale Baueinheit auszubilden, beispielsweise dadurch, dass die Kompressionshülle 4 an den Schlauch- oder Rohrkörper 2 angespritzt oder anextrudiert ausgebildet ist.

Die Kompressionshülle 4 verfügt über eine Luftaustrittsöffnung 14, die bevorzugterweise der Peniseintrittsöffnung 9 gegenüberliegend ausgebildet ist.

Der Schlauch- oder Rohrabschnitt 5 ist flexibel ausgebildet und besteht bevorzugterweise aus einem Wellen- oder Faltenschlauch, wie in Fig. 1 dargestellt. Als Material für den Schlauch- oder Rohrabschnitt 5 kommt insbesondere ein flexibles Kunststoffmaterial in Frage, das eine gewisse Knickfestigkeit bereitstellt.

Zur Evakuierung des vom Schlauch- oder Rohrkörper 2 bereitgestellten Volumenraums 3, das heißt zur Ausbildung eines Unterdrucks innerhalb des hohlen Schlauch- oder Rohrkörpers 2 dient eine an den Anschlussstutzen 12 anzuschließende Vakuumpumpe, die bevorzugterweise geräuscharm und batteriebetrieben ausgebildet ist.

Für ein bestimmungsgemäßes Anlegen der erfindungsgemäßen Vorrichtung wird der Penis 15 durch die zweite Öffnung 9 in die Kompressionshülle 4 eingeführt. Dabei wird der Penis 15 mittels eines an dem Schlauch- oder Rohrkörper 2 angelegten Unterdrucks in die Kompressionshülle 4 eingesogen, zu welchem Zweck diese über die Luftaustrittsöffnung 14 verfügt. Der Schlauch- oder Rohrkörper 2 saugt sich infolge des angelegten Vakuums am Penisschaft fest, wodurch ein vakuumdichtes Anliegen erreicht wird.

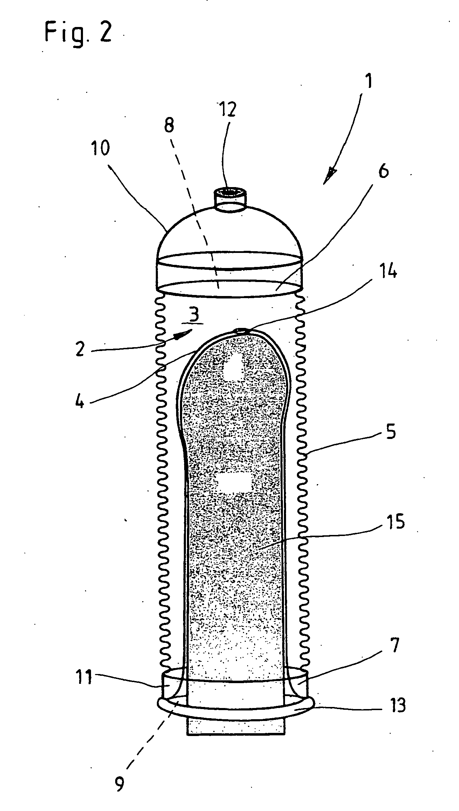

Fig. 1 zeigt eine Anwendungssituation, die einen zum Teil in die Kompressionshülle 4 eingeführten Penis 15 zeigt. Der Vorgang zur Ausbildung eines Unterdrucks im Schlauchoder Rohrkörper ist noch nicht abgeschlossen. Fig. 2 zeigt hingegen eine Anwendungssituation, gemäß welcher der Unterdruck bestimmungsgemäß aufgebaut ist. Der Penis 15 ist vollständig von der Kompressionshülle 4 aufgenommen und erstreckt sich in bestimmungsgemäßer Weise in den Schlauch- oder Rohrkörper 2 hinein.

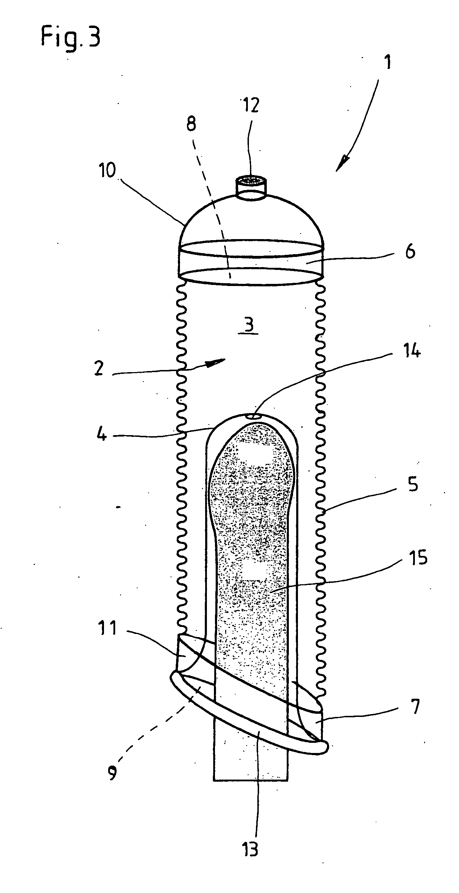

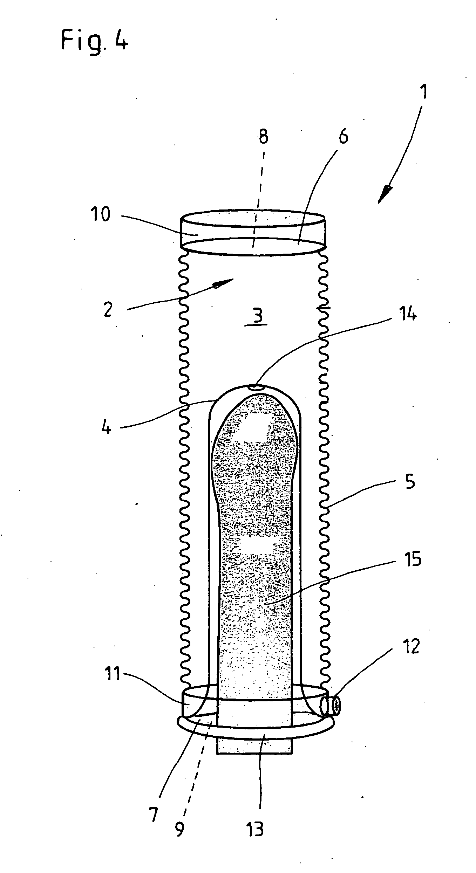

Die Fign. 3 und 4 zeigen in Entsprechung zur Darstellung nach Fig. 1 alternative Ausgestaltungsformen.

Die Ausführungsform nach Fig. 3 unterscheidet sich zu derjenigen nach Fig. 1 durch das abgeschrägt ausgebildete zweite Ende 7. Diese Abschrägung dient dazu, den Tragekomfort zu verbessern.

Die Ausgestaltung nach Fig. 4 zeigt eine Anordnung, dergemäß der Anschlussstutzen 12 nicht am Verschlusselement 6 sondern am materialverstärkten Ringabschnitt 11 angeordnet ist.

### Bezugszeichenliste

- 1: Vorrichtung
- 2: Schlauch- oder Rohrkörper
- 3: Volumenraum
- 4: Kompressionshülle
- 5: Schlauch- oder Rohrabschnitt
- 6: (erstes) Ende
- 7: (zweites) Ende
- 8: (erste) Öffnung
- 9: (zweite) Öffnung
- 10: Verschlusselement
- 11: Ringabschnitt
- 12: Anschlussstutzen
- 13: Ringwulst
- 14: Luftaustrittsöffnung
- 15: Penis

## Patentansprüche

1. Vorrichtung zur Penisextension durch langzeitliche Dehnungsbehandlung, mit einem einen evakuierbaren Volumenraum (3) bereitstellenden Schlauch- oder Rohrkörper (2) sowie mit einer der Penisaufnahme dienenden Kompressionshülle (4), die als innenliegende Hülle innerhalb des vom Schlauch- oder Rohrkörper (2) bereitgestellten Volumenraums (3) angeordnet ist, **dadurch gekennzeichnet, dass** der Schlauch- oder Rohrkörper (2) aus einem Kunststoffmaterial gebildet ist, das flexibel und gleichzeitig knickstabil geformt ist und wobei die Kompressionshülle (4) aus einem dehnungsfähigen Material, vorzugsweise Gummi, Kautschuk, Silikon und/oder dergleichen besteht.

2. Vorrichtung nach Anspruch 1, **dadurch gekennzeichnet, dass** der Schlauch- oder Rohrkörper (2) einen beidseitig offenen Schlauch- oder Rohrabschnitt (5) aufweist.

3. Vorrichtung nach Anspruch 2, **dadurch gekennzeichnet, dass** der Schlauch- oder Rohrabschnitt (5) einendseitig mittels eines Verschlusselements (10) vakuumdicht verschließbar ausgebildet ist.

4. Vorrichtung nach Anspruch 2 oder 3, **dadurch gekennzeichnet, dass** der Schlauchoder Rohrabschnitt (5) anderendseitig einen verstärkt ausgebildeten Ringabschnitt (11) aufweist.

5. Vorrichtung nach Anspruch 3 oder 4, **dadurch gekennzeichnet, dass** das Verschlusselement (10) oder der Ringabschnitt (11) einen Anschlussstutzen (12) zum Anschluss einer Vakuumpumpe aufweist.

6. Vorrichtung nach einem der vorhergehenden Ansprüche, **dadurch gekennzeichnet, dass** der Schlauch- oder Rohrkörper (2) eine Länge von ca. 120 % des erigierten Penis aufweist.

7. Vorrichtung nach einem der vorhergehenden Ansprüche, **dadurch gekennzeichnet, dass** die Kompressionshülle (4) auswechselbar am Ringabschnitt (11) des Schlauch- oder Rohrabschnitts (5) festlegbar ist.

8. Vorrichtung nach einem der vorhergehenden Ansprüche 1 bis 6, **dadurch gekennzeichnet, dass** die Kompressionshülle (4) und der Schlauch- oder Rohrabschnitt (5) als integrale Baueinheit ausgebildet sind.

9. Vorrichtung nach einem der vorhergehenden Ansprüche, **dadurch gekennzeichnet, dass** die Kompressionshülle (4) eine Luftaustrittsöffnung (14) aufweist, die bevorzugterweise der Peniseintrittsöffnung (9) gegenüberliegend ausgebildet ist.

10. Vorrichtung nach einem der vorhergehenden Ansprüche, **dadurch** gekenntzeichnet, dass die Kompressionshülle (4) eine Länge und einen Umfang aufweist, die der Länge und dem Umfang eines Penis im schlaffen Zustand entsprechen.

11. Vorrichtung nach einem der vorhergehenden Ansprüche, **dadurch gekennzeichnet, dass** der Schlauch- oder Rohrkörper (2) aus einem Wellen- oder Faltenschlauch gebildet ist.

12. Vorrichtung nach einem der vorhergehenden Ansprüche, **gekennzeichnet durch** eine mobile Vakuumpumpe.

## Claims

1. A device for penis extension through long-term extension treatment, having a tubular body (2) which provides an evacuable volume space (3) and having a compression sleeve (4) which serves to receive the penis and is arranged as an interior sleeve inside the volume space (3) provided by the tubular body (2), **characterized in that** the tubular body (2) is formed by a plastic material, which is flexible while still at the same time being shaped to be kink-resistant, and the compression sleeve (4) is made of a stretchable material, preferably rubber, silicone and/or the like.

2. The device according to Claim 1, **characterized in that** the tubular body (2) has a tubular section (5), which is open at both ends.

3. The device according to Claim 2, **characterized in that** the tubular section (5) is designed to be closable at one end by means of a closure element (10), so that it is vacuum-tight.

4. The device according to Claim 2 or 3, **characterized in that** the tubular section (5) has an annular section (11) designed with reinforcement at the other end.

5. The device according to Claim 3 or 4, **characterized in that** the closure element (10) or the annular section (11) has a connection nozzle (12) for connecting a vacuum pump.

6. The device according to any one of the preceding claims, **characterized in that** the tubular body (2) has a length of approximately 120% of the erect penis.

7. The device according to any one of the preceding claims, **characterized in that** the compression sleeve (4) is replaceably detachable to the annular section (11) of the tubular section (5).

8. The device according to any one of the preceding Claims 1 through 6, **characterized in that** the compression sleeve (4) and the tubular section (5) are designed as an integral modular unit.

9. The device according to any one of the preceding claims, **characterized in that** the compression sleeve (4) has an air outlet opening (14), which is preferably designed to be opposite the penis entrance opening (9).

10. The device according to any one of the preceding claims, **characterized in that** the compression sleeve (4) has a length and a circumference which correspond to the length and circumference of a penis in the flaccid state.

11. The device according to any one of the preceding claims, **characterized in that** the tubular body (2) is formed by a wavy or folded tube.

12. The device according to any one of the preceding claims, **characterized by** a mobile vacuum pump.

## Revendications

1. Dispositif d'extension de pénis par un traitement par dilatation de longue durée, comprenant un corps de flexible ou de tuyau (2) mettant à disposition un espace volumique (3) pouvant être vidé, ainsi qu'une gaine de compression (4) servant à recevoir le pénis qui est disposée en tant que gaine intérieure à l'intérieur de l'espace volumique (3) mis à disposition par le corps de flexible ou de tuyau (2), **caractérisé en ce que** le corps de flexible ou de tuyau (2) est fabriqué dans un matériau plastique qui est flexible et à la fois stable au pliage, et sachant que la gaine de compression (4) est composée d'un matériau pouvant se dilater, de préférence de l'élastique, du caoutchouc, de la silicone et/ou similaire.

2. Dispositif selon la revendication 1, **caractérisé en ce que** le corps de flexible ou de tuyau (2) présente une section de flexible ou de tuyau (5) ouverte des deux côtés.

3. Dispositif selon la revendication 2, **caractérisé en ce que** la section de flexible ou de tuyau (5) est formée en pouvant être fermée de manière étanche au vide à une extrémité au moyen d'un élément de fermeture (10).

4. Dispositif selon la revendication 2 ou 3, **caractérisé en ce que** la section de flexible ou de tuyau (5) présente sur l'autre côté une section annulaire (11) réalisée de manière renforcée.

5. Dispositif selon la revendication 3 ou 4, **caractérisé en ce que** l'élément de fermeture (10) ou la section annulaire (11) présente une tubulure de raccordement (12) pour raccorder une pompe à vide.

6. Dispositif selon l'une des revendications précédentes, **caractérisé en ce que** le corps de flexible ou de tuyau (2) présente une longueur d'environ 120% du pénis en érection.

7. Dispositif selon l'une des revendications précédentes, **caractérisé en ce que** la gaine de compression (4) peut être fixée de manière amovible sur la section annulaire (11) de la section de flexible ou de tuyau (5).

8. Dispositif selon l'une des revendications précédentes 1 à 6, **caractérisé en ce que** la gaine de compression (4) et la section de flexible ou de tuyau (5) sont formées en tant qu'unité intégrale.

9. Dispositif selon l'une des revendications précédentes, **caractérisé en ce que** la gaine de compression (4) présente une ouverture de sortie d'air (14), qui, de manière plus préférée, est formée face à l'ouverture d'entrée du pénis (9).

10. Dispositif selon l'une des revendications précédentes, **caractérisé en ce que** la gaine de compression (4) présente une longueur et une circonférence qui correspondent à la longueur et à la circonférence d'un pénis à l'état de repos.

11. Dispositif selon l'une des revendications précédentes, **caractérisé en ce que** le corps de flexible ou de tuyau (2) est formé d'un flexible ondulé ou à soufflets.

12. Dispositif selon l'une des revendications précédentes, **caractérisé par** une pompe à vide mobile.
